## Europäisches Patentamt
## European Patent Office
## Office européen des brevets

(19)

(11) Publication number: **0 012 718**
**B1**

(12) **EUROPEAN PATENT SPECIFICATION**

(45) Date of publication of patent specification: **16.09.81**

(51) Int. Cl.³: **A 61 K 39/10, C 12 N 1/20**

(21) Application number: **79810169.7**

(22) Date of filing: **30.11.79**

(54) Intra-respiratory vaccine, modified bacteria strain used in it, vaccine dose form and process for preparing it.

(30) Priority: **07.12.78 US 967477**
**07.12.78 US 967478**

(43) Date of publication of application:
**25.06.80 Bulletin 80/13**

(45) Publication of the grant of the European patent:
**16.09.81 Bulletin 81/37**

(84) Designated Contracting States:
**AT BE CH DE FR GB IT NL SE**

(56) References cited:
**US - A - 4 016 253**

MICROBIOLOGY ABSTRACTS, Section B, vol. 8, no. 3 March 1973, page 158, abstract no. 8B2465 D. L. HARRIS et al.:"Immunization of pigs against Bordetella bronchiseptica infection by parenteral vaccination".

MICROBIOLOGY ABSTRACTS, Section B, vol. 14, no. 5, May 1979, page 160, abstract no. 5448—B14
T. SHIMIZU: "Prophylaxis of Bordetella bronchiseptica infection in guinea pigs by intranasal vaccination with live strain ts-S34".

(73) Proprietor: **Iowa State University Research Foundation, Inc.,**
**315 Beardshear, Iowa State University Ames Iowa 50010 (US)**

(72) Inventor: **Switzer, William Paul**
**R.R. 1**
**Cambridge, Iowa 50046 (US)**
Inventor: **Farrington, Daniel Owen**
**218 Highland Road**
**Terre Haute, Indiana 47802 (US)**
Inventor: **Goodnow, Robert Allen**
**R.F.D. 4 Box 45A**
**Omaha, Nebraska 68137 (US)**
Inventor: **Shade, Floyd Jerry**
**16106 Ohern**
**Omaha, Nebraska 68137 (US)**
Inventor: **Sloboth, Thomas Anthony**
**5865 Gold Street**
**Omaha, Nebraska 68106 (US)**

(74) Representative: **Antony, Fritz, Dr. et al,**
**P.O. Box 601 Winkelriedstrasse 35**
**CH-6002 Lucerne (CH)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Intra-respiratory vaccine, modified bacteria strain used in it, vaccine dose form and process for preparing it

*Bordetella bronchispectica* is capable of infecting the nasal passages and respiratory tracts of many animals, particularly mammals. *B. bronchiseptica* is the cause of atrophic rhinitis and pneumonia in swine. Harris and Switzer, *Am. J. Vet. Res., 30,* 1161—1166 (1969). A major lesion of the disease in swine is commonly referred to as "turbinate atrophy" because following the primary *B. bronchiseptica* infection, the nasal turbinate bones frequently undergo serious deterioration. See Switzer and Ferrington U.S. patent 4,016,253 (1977).

In dogs, *B. bronchiseptica* has been characterized as the primary etiological agent in infectious canine tracheo-bronchitis more commonly known as kennel cough. Wright et al, *Vet. Rec.,* Nov. 3, 1973, 486—487; Appel et al, Cornell Research Laboratory for Disease of Dogs, Laboratory Report, Series 2, No. 6 (May, 1976). The latter publication states that kennel cough is a highly contagious respiratory disease of dogs which, although not life-threatening, should be prevented. The disease causes suffering to the dogs and is unpleasant for dog owners. It is commonly transmitted when dogs are placed in kennels for boarding.

Other mammalian species are also afflicted with *B. bronchiseptica* infections of the respiratory tract. These include laboratory animals such as guinea pigs, rabbits, and rats, as well as animals raised for meat or fur, such as rabbits and chinchilla. See Nakagawa et al, *Jap. J. Vet. Sci., 33(2),* 53—60 (1971); Oldenburg et al, *Monatshefte für Veterinärmedizin,* 27(19), 738—743 (1972); Burek et al, *Lab An, Sci.,* 22(6), 844—849 (1972); loakimidis et al, *Kteniatrika Nea Thessaloniki, 2,* 31—33 (1970). As with swine and dogs, the virulent *B.* bronchiseptica colonise the respiratory mucosa and thereby produce the clinical symtoms of the infection. *B. bronchiseptica* may also cause pheumonia in monkeys and other zoo animals. Graves, *Lab. An. Car., 20(2),* 246—250 (1970). Cats are carriers of *B. bronchiseptica* and may spread the disease to other animals, Fisk et al, *Lab. An. Sci. 23(1),* 33—35 (1973).

Switzer and Harris found that the introduction of live cells of a low-virulence strain of *B. bronchiseptica* into the nasal cavities of non-immune swine would cause a relatively mild infection, and that thereafter the swine would be immune to further infection, and would thereby be protected against turbinate atrophy. See *J. Vet. Res., 30,* 1161—1166 (July, 1969). The attenuated strain of *B. bronchiseptica* tested by Switzer and Harris was designated strain D—1. Although introduction of strain D—1 into the nasal passage of swine protected the swine against turbinate atrophy, it was found that strain D—1 persisted in the nasal passages and produced a mild damage to the nasal epithelium. There was no evidence of reversion to a swine virulent form of *B. bronchiseptica,* but it was feared that strain D—1 could infect other animals such as dogs. The need for an effective live intranasal vaccine which has no adverse effect and which does not persist on the nasal mucosa has not been met so far.

Parenteral vaccines for intramuscular injection are also known. For example, one was prepared from a virulent strain of *B. bronchiseptica* (identified as strain B), but this whole-cell vaccine failed to induce adequate resistance to nasal infection as reported by Harris and Switzer, *Am. J. Vet. Res., 30,* 1161—1166 (July, 1969). The cells were killed to form the bacterin.

U.S. patent 4,016,253 describes a parenteral vaccine for intramuscular injection prepared from killed whole-cells of strain D—1 (ATCC No. 31124).

The resistance to *B. bronchiseptica* infection induced by parenteral vaccines such as the strain D—1 vaccine is different than the immunity produced by intranasal infection of swine with live *B. bronchiseptica.* Parenteral vaccination does not prevent nasal infection by virulent *B. bronchiseptica,* although it does accelerate nasal clearance of the infection, and effectively protects against the development of the gross lesions associated with atrophic rhinitis, and the secondary destruction of the turbinate bones referred to as turbinate atrophy. Further, even a mild infection of the nasal passages of parenterally vaccinated swine may make the swine more susceptible to secondary infections of the respiratory tract.

The strain D—1 parenteral vaccine used for swine has not been found to be effective for immunizing dogs against *B. bronchiseptica.* There is an increase in the circulating antibody titer, but the dogs still contract the infection and manifest symptoms of kennel cough. Further, the parenteral injection of strain D—1 vaccines may have undesirable side effects, including swelling at the site of the injection, anophylactic shock, and other toxic effects on the dogs. Some investigators (McCanldish—1976 and Shelton—1977) have reported experimental immunization of dogs against *B. bronchiseptica,* infection with a parenteral vaccine. McCanldish et al.l *Vet. Rec.,* 98, 156—157 (1976); Shelton et al, *Vet. Med./Small Animal Clinician,* February, 1977, 189—193. However, another investigator (Appel—1977) has been unsuccessful in repeating the McCanldish or Shelton work. See Bemis, Greisen, and Appel, *J. Infec. Disease,* 135, 753—758 (May, 1977).

In 1978 Shimizu reported on experiments with guinea pigs using an intra-nasal vaccine prepared from a mutated temperature-sensitive strain of *B. bronchiseptica.* Infect. Immun., *22(2),* 318—321 (Nov. 1978). The temperature sensitive strain ts-S34, which is described as having favorable properties for potential use as a live attenuated vaccine, was able to grow at 32°C but unable to grow at 34°C. Since the temperature of nasal turbinate mucosa of swine is said to be approximately 32 to 34°C, it was found in the guinea pig test that the mutated strain at first grew in large numbers in the nasal turbinates and then decreased. However, the data did not show a self-clearing action from $10^3$ to $10^4$ cells per gram of tissue being found four to six weeks after inoculation. Further, although strain ts-S34 had less tendency to revert to a virulent or wild form of B. bronchiseptica than another mutated strain, there was still a significant reversion. Commercially acceptable vaccines are not known to have been prepared from the Shimizu strain ts-S34.

The present invention provides an intra-respiratory vaccine for animals subject to Bordetella bronchiseptica infection which is characterized by being an aqueous suspension of viable cells of the modified Bordetella bronchiseptica strain identified as ATCC strain No. 31437 or of a strain derived therefrom and having essentially the same characteristics for intra-respiratory vaccine use, said aqueous suspension containing at least $2 \times 10^3$ of said viable cells per milliliter. On October 2, 1978 there has been placed on deposit with the American Type Culture Collection, Rockville, Md., viable samples of strain 55 B. bronchiseptica, which are now freely available upon request. To this strain has been assigned the ATCC No. 31437, which number will therefore also be used herein as an alternative designation to strain 55.

Strain 55 was isolated in 1955 from the pneumonic lungs and normal nasal cavity of an experimental pig inoculated intranasally with crude pneumonic swine lung suspension. The initial inoculum was secured from a pig in a herd experiencing clinical atrophic rhinitis and pneumonia. This organism was seeded into rubber stoppered 100 milliliter vials of tryptose phosphate broth (TPB) in 1955 and incubated at 37°C. These vials were then committed to a duration of survival trial. The cultures were sampled several times during the ensuing years and found to be viable,. In January or 1972, after 17 years without serial passage, contents of a vial were cultured on 5% horse blood agar. Two extremely rough bacterial colonies developed and one colony was picked into TPB. This organism was subsequently identified as a highly modified B. bronchiseptica and designated as strain 55.

In its taxonomic characteristics strain 55 is distinguished from a virulent strain of B. bronchiseptica (strain B) and from an attenuated strain (strain D—1) in several aspects:

(1) Strain 55 colonies on modified MacConkey's agar medium without Furaltadone incubated at 37°C has a "lacy" margin (and therefore is not temperature sensitive). Strain D—1 and B colony margins were undulate. The colony diameter at 48 hours was approximately 1 mm. with colonies up to several millimeters in diameter present after prolonged incubation. All 3 strains formed colonies on 5 percent horse blood agar that were circular, smooth, opaque, and homogenous with an entire edge. The colony size and morphology of the B. bronchiseptica strain varied greatly depending on the concentration of organism on the culture plate, hours (days) of incubation at 37°C or room temperature and the relative humidity under which the culture plates were maintained. Aged colonies on modified MacConkey's agar without Furaltadone were characterized by central collapse of the colony and pronounced undulating rays from the cell margin giving the colony a "wagon wheel" shape.

(2) Strains D—1 and B were motile while 55 strain was often nonmotile when examined by the hanging drop method.

(3) Strain 55 was sensitive to Furaltadone at the 0.02 mg./ml. concentration in the modified MacConkey's medium while D—1 and B strain were not.

(4) Strain 55 gave a slow and weak positive reaction (<24 hours) on Simmon's citrate agar slants while D—1 and B strains were strongly positive at 18 to 24 hours.

(5) Strain 55 had the highest hemagglutinating titer among the 3 strains.

(6) Strain 55 has two plasmids, one of 31 megadaltons and one of 3 megadaltons. In addition, the d2 cell membrane protein is greater in amount than other strains, such as strain B (virulent) and strain D—1 (avirulent).

When viable cells of strain 55 are deposited in sufficient numbers in the nasal passages of swine, they multiply and form colonies in the mucous membranes of the nasal passages. The colonies persist for only a few days, and are usually cleared from the nasal passages of swine in less than a week. When the strain 55 cells are applied to the nasal or other respiratory mucosa of dogs, colonies at first form and are then cleared. With dogs the colonies persist for a somewhat longer time, several weeks being required for clearance. During the time in which the colonies of strain 55 are present in the respiratory mucosa of the animals, they do not produce any clinical symptoms of disease. However, the resistance of the animals to subsequent infection by virulent B. bronchiseptica is significantly increased. The local immunization inhibits the growth of infectious B. bronchiseptica, and greatly accelerates the clearance rate. In swine, clinical symptoms of atrophic rhinitis are prevented, and the subsequent atrophy of the turbinate is greatly reduced. In dogs, clinical symptoms of kennel cough (tracheobronchitis) are prevented.

In preparing the vaccine of the present invention, viable cells of Bordetella bronchiseptica strain ATCC No. 31437, which may have been subjected to freeze-drying for preservation, are introduced into a suitable culture medium, which is then incubated at a temperature favouring the growth of the organism. In general, published procedures for culturing B. bronchiseptica organisms are employed. See, for example, Am. J. Vet. Res., 30, 1161, 1162 (1969); and Am. J. Vet. Res., 33, 975, at 1976 (1972). More specifically, tryptose phosphate broth (TPB) may be used for propagation of the organism. One suitable source of such a TPB medium is Difco Laboratories, Inc., Detroit, Mich. Other useable culture mediums include: Bordet-Gengou Agar (Difco), Brain-Heart Infusion Broth (Difco), tryptone soya broth (Osoid Limited, London, England). Propagation temperatures of 36° to 38° are favorable.

The cultured cells are preferably recovered (harvested) without concentration by centrifugation or filtration. Since the cells are to be used live, it is desirable to avoid damage to the cells by mechanical processing. Cell cultures having a sufficiently high concentration of the strain 55 cells are obtainable, and the residual fermentation nutrients can remain with the cells for a vaccine administration. In general, the cell culture after fermentation should have a concentration of at least $1 \times 10^7$ viable cells per milliliter and may range from $1 \times 10^7$ to $1 \times 10^9$ cells/ml.

For vaccine use, strain 55 cells are employed as an aqueous suspension, which can be readily administered to the animals by application to the nasal mucosa. For example, the aqueous suspension of the cells may be introduced into the nasl passages in a measured amount by means of a syringe, or a measured amount of the aqueous cell suspension may be sprayed into the nostrils. The cell suspension should therefore be sufficiently liquid so that it is readily administrable or sprayable. Depending on the animals with which the vaccine is to be used and the amount of the cell suspension to be intranasally administered, the concentration of viable cells may vary over a wide range. For exampe, concentrations of from $2 \times 10^3$ to $10^8$ cells per milliliter can be used. Even with small animals such as guinea pigs and rabbits, however, it will usually be desirable to administer at least one thousand viable cells per nostril, making a total dose of $2 \times 10^3$ cells. Larger doses for swine and dogs are desirable, such as at least $1 \times 10^5$ viable cells per milliliter. Usually, the optimum dose will be within the range from $1 \times 10^6$ to $1 \times 10^8$ cells per milliliter.

While a degree of immunization may be obtained by introducing the vaccine into one nostril of the animal or elsewhere in the respiratory tract, the preferred procedure for swine is to introduce approximately equal amounts of the aqueous cell suspension into both nostrils (nares). The dose volume tends to be limited by the amount of liquid that the nostrils can retain.

For the purposes of the present invention, cell counts may be made by standard procedures. The cell concentrations per milliliter of the vaccine are determined by plating the cells and counting the CFU (colony forming units). The propagation for determining CFU may be on standard plates, such as 5% horse blood agar, T.P.B. agar of MacConkeys agar.

For preparing a vaccine dose form, which may be stored, shipped, and prepared for administration at the time of the intra-respiratory vaccination the cell suspension obtained by fermentation is subjected to freeze-drying (lyophylization). There is added to the cell suspension prior to freeze-drying, microbiological cryoprotectants and stabilizers, as is known in the art. The cryoprotectant-stabilizer means are added as an aqueous solution, which thereby dilutes the cell concentration. For example, a fermentation cell density of $2 \times 10^8$ cells/ml. may be reduced to $1.3 \times 10^8$ cells/ml. Also, and despite the addition of cryoptrotectants, the freeze-drying of the cells will cause a reduction in the content of viable cells. Such loss of viability is inherent in freeze-drying of cells. For example, with strain 55 cells, a cell concentration after addition of the cryoprotectant-stabilizer of $1.3 \times 10^8$ may be reduced during dessication to $2 \times 10^6$. In addition, further loss of cell viability may occur during storage of the freeze-dried cells prior to use.

In accordance with the present invention, after freeze-drying, a measured quantity of the cells is introduced into a vaccine vial for storage and transport. The vial should contain at least one dose of the vaccine, and may contain individual or multiple doses. In general, each vaccine dose should provide at least $1 \times 10^6$ viable cells, such as a dose in the range of $1 \times 10^6$ to $1 \times 10^8$ viable cells.

Prior to administration of the vaccine sterile water is added to the cells in an amount appreciably less than the volume of water removed from the cells by freeze-drying. Preferably, the amount of water added to a quantity of the cells is at least 25% less than the volume of water removed from the cell quantity by the freeze-drying while being sufficient to form an aqueous suspension of the cells for the intra-respiratory administration. For example, the added sterile water may be equal to 40 to 60% of the water removed from the quantity of cells. In this way, the concentration of viable cells may be increased, thereby offsetting the effects of the cell dilution and viability loss.

The sterile water used for reconstituting the cells preferably contains a wetting agent for promoting the rehydration and the mucosal implantation of the cells. The wetting agent and the amount present should be non-inhibitory with respect to the growths of the cells. Cationic wetting agents are therefore, in general, not suitable because they have a bacteriostatic action. Non-ionic wetting agents are preferred, but non-inhibitory anionic wetting agents can also be used. The non-ionic wetting agent may be Triton X 100 (registered trade mark for octyl phenoxy polyethoxyethanol), available from Rohm & Haas Company, Philadelphia, Pennsylvania. It has been found that very low concentrations of this wetting agent are effective, such as concentrations in the sterile water of 0.01%. Other non-ionic wetting agents which may be used include Neodol (registered trade mark for ethoxylates containing 9—12 carbon atoms), available from Shell Chemical Company, Houston, Texas. Useable anionic wetting agents of very dilute concentrations include sodium lauryl sulfate, tallow alkyl sulfate, and sodium dodecyl sulfate. Other commercially available anionic wetting agents include Orvus AB Granules (registered trade mark for linear alkane sulfonates) obtainable from Proctor & Gample Company, Cincinnati, Ohio and Conco—AAS—35 (registered trade mark for 35% sodium dodecyl benzene sulfonate) obtainable from Continental Chemical Co., Clifton, New Jersey. These wetting agents may be used at very low concentrations for the purpose of the present invention, such as concentrations of 0.01% or less. For other non-ionic or anionic wetting agents that can be used for the purposes of this invention, reference may be made to the Surfactant Science Series: Nonionics (1967); and Anionics (1976); published by Marcel Dekker, Inc., New York. N.Y.

The mucous membranes of the respiratory tract tend to be resistant to the implantation of the cells. The ciliated epithelia of the upper respiratory tract will respond by trying to clear the cells from the nasal passages. There is also a tendency to clear foreign material from the mouth, throat and trachea. For effective use, the respiratory vaccine must possess the capability to resist this clearance. It is believed that the incorporation of a wetting agent in the sterile water used to form the vaccine suspension can help to resist clearance of the cells and to promote their implantation in the mucosa. The wetting agent will reduce the surface tension of the mucosal fluids flowing over the respiratory mucosa, thereby achieving a more rapid and closer contact of the viable cells with the mucosal tissue. In this way, the establishment of the cells and their colonization of the mucosal tissue is promoted, and it is this colonization which produces the local immunity to subsequent infection with virulent (pathogenic) *B. bronchiseptica*.

Where the vaccine is applied intranasally, as preferred for swine, it is desirable to introduce the vaccine into each nare. The total vaccine dose (both bares) may range from $1 \times 10^6$ to $1 \times 10^8$, and this amount of cells may be administered in from 0.7 to 1.3 milliliters. For example, a vaccine dose of approximately 0.5 milliliters per nare may be used at the concentration of about $4 \times 10^6$ viable cells per milliliter, thereby $2 \times 10^6$ cells being adminstered to the mucosa in each nostril.

A similar adminstration procedure may be used for dogs, that is, intranasal adminstration. However, it is believed that the easiest and most effective means for using the vaccine with dogs is by intra-pharynceal administration. In clinical kennel cough, the infection is found in the throat of the dog, particularly in the trachea. It has been found that applying a small amount of the cell suspension, such as 0.5 to 1.5 ml. to the upper portion of the pharynx (nasopharynx) can effectively result in colonization of the mucosa of the pharynx and trachea. The desirable cell densities are the same as for intranasal administration, that is, from about $1 \times 10^6$ to $1 \times 10^8$ cells per milliliter. For example, a concentration of $4 \times 10^6$ is satisfactory. It is believed that the vaccine for both intranasal and intrapharyngeal administration should contain at least $1 \times 10^6$ viable cells per milliliter.

The preparation of the intra-respiratory *B. bronchiseptica* vaccines can be practiced conveniently with a 2-vial packaging system. One vial will contain a measured quantity of the freeze-dried cells, and may contain a single or multiple dose quantity. For example, the vial may contain from $1 \times 10^6$ to $1 \times 10^8$ viable cells per dose. A single dose vial, therefore, as an example, might contain $4 \times 10^6$ viable cells. The other vial will contain the sterile water which preferably has the wetting agent dissolved therein, such as 0.01% concentration Triton X 100. The amount of water in the second vial will be substantially less than the water removed by freeze-drying. For example, the second vial may contain 1 ml. of water for reconstitution of the freeze-dried cells in the first vial from which 2 ml. of water were removed in the freeze-drying. More generally, as already indicated, the water in the second vial may equal 40 to 60% of the water removed during the freeze drying of the cells contained in the first vial. The volume of water to be used should be sufficient to form an aqueous suspension of the cells. Usually, the amount of water will be within the range from 0.7 to 1.3 ml. per cell dose.

The aqueous cell suspension, comprising the vaccine dose, may be administered by a syringe-type applicator, or by spraying the aqueous suspension onto the mucosa. For example, using a standard sterile 1 ml. plastic syringe, 1 ml. of the reconstituted vaccine may be drawn into the syringe, care being taken that the cells have been completely reconstituted and suspended before being filled into the syringe. Using an elongated plastic nasal applicator over the barrel of the syringe, the tip of the applicator may be inserted into the nares for application to the nasal mucosa, or into the mouth for application to the pharyngeal mucosa. For intranasal administration, half of the syringe volume (0.5 ml.) will be introduced into each side of the nose, while the entire contents (1 ml.) will be discharged onto the upper portion of the pharynx. Alternatively, the aqueous suspension may be applied with known intranasal or intra-oral atomizers. Using atomizer application, the amounts of the vaccine to be employed can be the same as for syringe application, but, if desired, a larger quantity of the vaccine may be applied.

The cryoprotectants and stabilizers useable for the purpose of the present invention are those well known in the art of bacterial cell preservation. They may also include nutrients. The substances used as cryoprotectants/stabilizers/nutrients for bacterial cells include dextran, gelatin, lactose, sodium glutamate, bovine albumin, digested casein, glutathione and phosphate salts, such as potassium hydrogen or dihydrogen phosphate.

The vaccine of this invention and the results which can be obtained by their use are further illustrated by the following examples.

Example I

Strain 55 *B. bronchiseptica* (ATCC No. 31437) was grown into tryptose phcsphate broth for 24 hours at 37°C. The culture was agitated by magnetic spin bar, or by the introduction of sterile air during the growth phase. A cell density of $2 \times 10^8$ cell/ml was obtained, and was considered

acceptable harvest culture. the culture was then formulated with a cryoprotectant-stabilizer system, such as solubilized caseinlactose formulation, a dextran-sugar combination, glycerol, or a serum-sugar combination. In this experiment, a 3-part formulation was prepared using sterile deionized water as follows:

| Solution I | Grams per Liter |
|---|---|
| N—Z Amine AS | 150.0 |
| Dextran | 60.0 |
| Gelatin | 4.0 |

| Solution II | Grams per Liter |
|---|---|
| Lactose | 200.0 |
| Sodium Glutamate | 4.0 |
| $KH_2PO_4$ | 2.12 |
| $K_2HPO_4$ | 5.0 |
| Bovine Albumin Fraction V | 20.0 |

| Solution III | |
|---|---|
| Glutathione | 50.0 |

The N—Z Amine As used in Solution I is a pancreatic digest of casein, which forms clear solutions in water. It is obtainable from Sheffield Chemical, Norwich, New York. Then Bovine Albumin Fraction V used in Solution II is prepared from bovine plasma and forms chemical solutions in water. It is obtainable from Miles Research Products, Miles Laboratories, Inc., Elkhart, Indiana.

Solution I was sterilized by autoclaving, and Solutions II and III were sterilized by sterile filtration. The solutions were then combined with the strain 55 cell culture in the following proportions:

| | Volume (milliliters) |
|---|---|
| Strain 55 culture ($2 \times 10^8$ cells/ml) | 6000 |
| Solution I | 1500 |
| Solution II | 1500 |
| Solution III | 29.7 |

The vaccine was then filled into sterile vaccine vials at 2 ml. fill volume (1.8 to 2.2 ml. vaccine volume range). The vialed vaccine was then frozen at —50°C, and thereafter dessicated in a commercial freeze-dryer. The viable cell count after drying was approximately $2 \times 10^6$ cell/milliliter so that each vial contained about $4.0 \times 10^6$ cells. Cell dilution from addition of the solutions I, II and III was from about $2 \times 10^8$ to $1 \times 10^8$/ml. The loss of viable cells from dessication was from about $1.3 \times 10^8$ to $2 \times 10^6$.

A second series of vials is filled with sterile deionized water containing 0.01% Triton X 100, a non-ionic wetting agent (octyl phenoxy polyethoxyethanol) obtained from Sigman Chemical Company, St. Louis, Missouri. Each vial was filled with 1 ml. of the sterile water containing the non-ionic wetting agent. In preparing the vaccine for intra-respiratory administration to swine or dogs, the contents of the two vials are combined, the 1 ml. of sterile water being added to the individual dose of the freeze-dried cells ($4 \times 10^6$ cells) providing an aqueous suspension having a volume of approximately 1 ml. with a

6

# 0012718

cell concentration of $4 \times 10^6$/ml. The reconstituted vaccine dose is then administered intranasally to dogs, 0.5 ml. being introduced into each nostril by syringe or atomizer, or administered intrapharyngeally for dogs, the 1 ml. suspension being introduced into the upper portion of the pharynx by syringe or aerosol.

### Example II

Strain 55 vaccine was prepared and reconstituted as described in Example I. The reconstituted vaccine was then tested with dogs. Ten dogs were used in the experiment, the dogs being free of *B. bronchiseptica* and non-immune thereto. The dogs were divided into two groups of five dogs each. The control group (Group I) was maintained in a separate facility while the treated dogs (Group II) were immunized. The freshly reconstituted vaccine was shaken vigorously to be sure that the dried vaccine pellet was fully dissolved and suspended. Each of the five dogs in Group II were inoculated in each nostril with 0.5 ml. of reconstituted vaccine using a syringe with a nasal applicator. The dogs thereby received approximately $2 \times 10^6$ viable cells of strain 55 per nostril. All of the dogs of both groups, on the fourteenth day after the immunizing of Group II were challenged with a virulent strain of *B. bronchiseptica*. The challenge culture contained approximately $2 \times 10^8$ cells/ml. Each test dog's nose was placed in a one cubic foot plastic box and exposed to air saturated with the virulent *B. bronchiseptica* for 2.5 minutes. All ten dogs were then housed in the same pen.

Thereafter, each dog was observed daily for clinical signs of kennel cough, and each dog was also nasal swabbed twice weekly for 40 days post-challenge.

None of the dogs of Group II (the immunized dogs) showed any clinical signs of kennel cough, while two of the dogs of Group III (the control dogs) demonstrated clinical signs of kennel cough. There was a highly significant suppression of the virulent challenge strain of *B. bronchiseptica* in the Group II dogs, as compared with the Group I dogs. To the extent that strain 55 cells remained in the nostrils of the Group II challenge, they were distinguished from the virulent challenge strain. The nasal swabs were cultured and bacterial counts of the virulent challenge cells were made in terms of CFU's (colony forming units). The results are summarized in Table A.

### TABLE A

#### *Average No. Organisms\*-Dog*

| Days Post Challenge | Vaccinates (CFU's) | Controls (CFU's) | Statistical % Difference |
|---|---|---|---|
| 6 | $1.8 \times 10^1$ | $2.0 \times 10^3$ | 99.1% |
| 16 | $3.0 \times 10^1$ | $2.0 \times 10^3$ | 98.5% |
| 21 | $7.6 \times 10^3$ | $1.0 \times 10^6$ | 99.2% |
| 34 | $2.2 \times 10^2$ | $1.9 \times 10^6$ | 99.9% |

\*Virulent challenge strain of *B. bronchiseptica*

### Example III

Three milliliters of sixth passage strain 55 modified *B. bronchiseptica* 24-hour tryptose phosphate broth (TPB) culture was inoculated into cotton-stoppered flasks containing 50 ml. of TPB and incubated at 37°C for 24 or 48 hours. The live seventh passage TPB cultures of strain 55 were used for intranasal vaccination of swine. Twenty-four hour cultures were administered for the first dose and forty-eight hour cultures were used for the second and subsequent doses. The 24-hour strain 55 seventh passage TPB culture contained approximately $1 \times 10^6$ organisms/ml. and the 48-hour TPB culture approximately $1 \times 10^7$ organisms/ml. These titers were determined as colony forming units (CFU) on 5% horse blood agar. The vaccines were administered on the day of their preparation.

A dosage of 0.5 ml. per nostril of the 24 and 48 hour TPB cultures in the form of nosedrops was administered to each intranasally immunized pig. The tip of a 5 milliliter syringe was inserted into each nostril and used to instill the TPB culture into the nasal cavity. Administration of the culture was timed with the inspiration of the pig.

The ability to live, low-virulence strain 55 *B. bronchiseptica* intranasal vaccination to produce nasal resistance to subsequent challenge with a swine-virulent *B. bronchiseptica* (strain B) was tested. The nasal persistence of strain 55 infection was also evaluated.

Thirteen pigs, 4-to-8-weeks of age, were obtained from rhinitis free herds. All pigs were culture-negative for *B. bronchiseptica*. The pigs were selected into two groups and housed in individual isolation units.

7

# 0012718

Six pigs received 3 doses over a 4 day period of live, low-virulence strain 55 intranasal vaccine at 7 weeks of age and seven pigs served as non-vaccinated controls. All pigs were challenged with virulent *B. bronchiseptica* (strain B) 3 weeks post-intranasal vaccination. Strain 55 was not detected by the nasal swab culture method in the nasal secretions of the vaccinated pigs 2 weeks post-intranasal vaccination.

A summary of the incidence of *B. bronchiseptica* in the nasal secretions of the B strain challenged pigs is presented in Table B. At 3 weeks post-B strain challenge 0 of 6 vaccinates and 5 of 7 control pigs were culture-positive for *B. bronchiseptica*. At 4 weeks post-B strain challenge the nasal secretions of 3 of 6 vaccinated pigs were culture-positive for *B. bronchiseptica* with an average colony count of 6 organisms per culture plate. The 8 week termination figures were 0 of 6 and 7 of 7 respectively. The average termination PAST serum titer was 39 for the vaccinated pigs and 53 for the non-vaccinated controls. At necropsy, gross examination revealed all intranasally immunized pigs had normal nasal turbinates while 2 of 3 controls examined had moderate to severe turbinate atrophy.

The procedure for the determination of gross turbinate atrophy was as follows: A cross section of the nasal cavity was made at the level of the second premolar tooth. Gross distortion or atrophy of the nasal turbinates and nasal septal defects were characterized depending on severity as mild, moderate or severe.

## TABLE B

### Number of swine culture-positive for
### *B. bronchiseptica* over number sampled

| Swine/product | Number of Swine | Pretrial | Weeks after virulent B strain *B. bronchiseptica* challenge | | | | Gross evidence of Turbinate Atrophy at necropsy |
|---|---|---|---|---|---|---|---|
| | | | 3 | 4 | 6 | 8 | |
| 4 to 8-week old pigs live low-virulence strain 55 intranasal vaccination | 6 | 0/6 | 0/6 | 3/6 | 0/6 | 0/6 | 0/6[a] |
| 4 to 8-week old pigs non-vaccinated controls | 7 | 0/7 | 5/7 | 6/7 | 6/7 | 7/7 | 2/3 |

[a] Number of pigs showing gross turbinate atrophy over number necropsied.

## Example IV

There is no evidence to indicate the persistence in or transfer between swine of strain 55 *B. bronchiseptica*. In the absence of demonstrable swine to swine transfer for the determination of potential increased virulence of strain 55, the organism was back passaged through 11 passages in 6-to-8-day old embryonating hens eggs and introduced into the nasal cavities of susceptible 3-week-old pigs. Strain 55 *B. bronchiseptica* killed 100 percent of the embryos by 72 hours in the first passage. Swine virulent B strain *B. bronchiseptica* regularly kills 100 percent of 6-to-8-day-old chicken embryos by 24 hours post-yolk sac inoculation. An increase in the virulence (earlier mortality) of 55 strain for chicken embryos was not evidenced through 11 serial passages. Strain 55 eleventh passage egg yolk inoculum was not detected in the nasal secretions of the intranasally inoculated pigs at 1 or 2 weeks post-inoculation.

**Claims for the Contracting States: BE, CH, DE, FR, GB, IT, NL, SE**

1. An intra-respiratory vaccine for animals subject to *Bordetella bronchiseptica* infection, characterized by being an aqueous suspension of viable cells of the modified *Bordetella bronchiseptica* strain identified as ATCC strain No. 31437 or of a strain derived therefrom and having essentially the same characteristics for intra-respiratory vaccine use, said aqueous suspension containing at least $2 \times 10^3$ of said viable cells per milliliter.

2. The vaccine of claim 1 in which said aqueous suspension contains at least $1 \times 10^5$ of said viable cells per milliliter and also contains residual culture nutrients.

3. The vaccine of claim 1 or 2 in which said aqueous suspension is sprayable and contains $1 \times 10^6$ to $1 \times 10^8$ of said viable cells per milliliter.

4. A vaccine dose form of an attenuated avirulent strain of *Bordetella bronchiseptica* for implant colonization of the respiratory mucosa, comprising a combination of a first vial containing freeze-dried viable cells of *B. bronchiseptica* ATCC strain No. 31437 or of a strain derived therefrom and having essentially the same characteristics for intra-respiratory vaccine use, said first vial containing at least one dose of said vaccine, and of a second vial containing sterile water for addition to the cells of said first vial to form an aqueous suspension thereof, said aqueous suspension formed by adding said sterile water to said cells having a concentration of at least $1 \times 10^6$ of said viable cells per milliliter.

5. The vaccine dose form of claim 4, wherein the second vial contains a wetting agent dissolved in the sterile water, said wetting agent being present in an effective amount for promoting the rehydration of the cells and the mucosal implanting thereof, said wetting agent being non-inhibitory to the growth of said cells.

6. The vaccine dose form of claim 5, wherein said wetting agent is an anionic or non-ionic wetting agent.

7. The vaccine dose form of any one of claims 4 to 6 wherein the amount of said sterile water in said second vial has a volume of from 40 to 60% of the volume of water removed from the cells in said first vial in freeze-drying, the amount of said viable cells in said first vial being a single dose of said vaccine of from $1 \times 10^6$ to $1 \times 10^8$ cells, and the amount of water in said second vial forming said aqueous suspension at a concentration of from $1 \times 10^6$ to $1 \times 10^8$ viable cells per milliliter.

8. A process of preparing a vaccine dose form of an attenuated avirulent strain of *Bordetella bronchiseptica* for implant colonization of the respiratory mucosa, characterized in that a culture of *B. bronchiseptica* strain ATCC No. 31437 or of a strain derived therefrom and having essentially the same characteristics for inter-respiratory vaccine use is cultured in an aqueous nutrient medium to obtain an aqueous suspension of viable cells having a concentration of at least $1 \times 10^7$ viable cells per milliliter, an aqueous solution of microbiological cryoprotectant and stabilizer means is added to said cell suspension with consequent dilution of the cells concentration, the diluted cell suspension is subjected to freeze-drying the resulting dried cells having a reduced content of viable cells because of the loss of viability resulting from freeze-drying, a measured quantity of said freeze-dried cells is introduced into a vaccine vial for storage and transport of the thus-prepared vaccine said vial containing at least one dose of said vaccine, and prior to administration of said vaccine sterile water is added to said quantity of cells, the amount of said added water being at least 25% less than the volume of water removed from said cell quantity by said freeze-drying while being sufficient to form an aqueous suspension of said cells for intra-respiratory administration.

9. The process of claim 8, characterized in that said added sterile water contains an effective amount of a wetting agent for promoting the rehydration of said cells and the mucosal implanting thereof, said wetting agent in the amount present being non-inhibitory to the growth of said cells.

10. The modified strain of *Bordetella bronchiseptica* identified as ATCC strain No. 31437 or a strain derived therefrom and having essentially the same characteristics for intra-respiratory vaccine use, capable to clear from the nasal passages of swine and dogs after colonization thereof.


**Revendications pour les Etats contractants BE, CH, DE, FR, GB, IT, NL, SE**

1. Vaccin intra-respiratoire pour animaux sujets à une infection de *bordetella bronchiseptica*, caractérisée en ce qu'il a la forme d'une suspension aqueuse de cellules viables de la souche modifiée de *bordetella bronchiseptica* identifiée par souche ATCC n° 31437 ou d'une souche qui en est dérivée et ayant essentiellement les mêmes caractéristiques pour un usage comme vaccin intra-respiratoire, ladide suspension aqueuse contenant au moins $2 \times 10^3$ cellules viables par millilitre.

2. Vaccin selon la revendication 1, caractérisé en ce que la suspension aqueuse précitée contient au moins $1 \times 10^5$ cellules viables par millilitre et contient également des substances nutritives résiduelles de la culture.

3. Vaccin selon l'une quelconque des revendications 1 ou 2, caractérisé en ce que la suspension aqueuse précitée est pulvérisable et contient $1 \times 10^6$ à $1 \times 10^8$ cellules viables par millilitre.

4. Forme de dose de vaccin d'une souche avirulente atténuée de *boredetella bronchiseptica* pour colonisation par implant de la muqueuse respiratoire, caractérisée en ce qu'elle comprend une combinaison d'une première fiole contenant des cellules viables lyophilisées de la souche ATCC n° 31437 de *B. bronchiseptica* ou d'une souche qui en est dérivée, et ayant essentiellement les mêmes caractéristiques pour un usage pour un vaccin intra-respiratoire, ladite première fiole contenant au moins une dose dudit vaccin, et d'une seconde fiole contenant de l'eau stérile pour addition aux cellules de ladite première fiole afin d'en former une suspension aqueuse, ladite suspension aqueuse formée par addition de ladite eau stérile auxdites cellules ayant une concentration d'au moins $1 \times 10^6$ cellules viables par millilitre.

5. Forme de dose de vaccin selon la revendication 4, caractérisée en ce que la seconde fiole précitée contient un agent mouillant dissous dans l'eau stérile, ledit agent mouillant étant présent en une quantité efficace pour favoriser la réhydratation des cellules et leur implantation dans la muqueuse, ledit agent mouillant n'inhibant pas la croissance desdites cellules.

6. Forme de dose de vaccin selon la revendication 5, caractérisée en ce que l'agent mouillant précité est un agent mouillant anionique ou non-ionique.

7. Forme de dose de vaccin selon l'une quelconque des revendications 4 à 6, caractérisée en ce que la quantité d'eau stérile dans la seconde fiole précitée représente un volume de 40 à 60% du volume de l'eau retirée des cellules dans la première fiole précitée lors de la lyophilisation, la quantité desdites cellules viables dans ladite première fiole étant une seule dose dudit vaccin de $1 \times 10^6$ à $1 \times 10^8$ cellules, et la quantité d'eau dans ladite seconde fiole formant la suspension aqueuse à une concentration de $1 \times 10^6$ a $1 \times 10^8$ cellules viables par millilitre.

8. Procédé de préparation d'une forme de dose de vaccin d'une souche avirulente atténuée de *bordetella bronchiseptica* pour colonisation par implant de la muqueuse respiratoire, caractérisé en ce qu'une culture de souche ATCC n° 31437 de *B. bronchiseptica* ou d'une souche qui en est dérivée et ayant essentiellement les mêmes caractéristiques pour un usage de vaccin intra-respiratoire, est mise en culture dans un milieu nutritif aqueux pour obtenir une suspension aqueuse de cellules viables ayant une concentration d'u moins $1 \times 10^7$ cellules viables par millilitre, une solution aqueuse d'un moyen stabilisant et cryoprotecteur microbiologique est ajoutée à ladite suspension de cellules avec dilution conséquente de la concentration des cellules, la suspension diluée des cellules est soumise à une lyophilisation, les cellules séchées résultantes ayant une teneur réduite en cellules viables du fait de la perte de viabilité résultant de la lyophilisation, une quantité mesurée desdites cellules lyophilisées est introduite dans une fiole de vaccin pour stockage et transport du vaccin ainsi préparé, ladite fiole contenant au moins une dose dudit vaccin, et avant administration, de l'eau stérile est ajoutée à ladite quantité de cellules, la quantité de ladite eau ajoutée représentant au moins 25% de moins que le volume de l'eau retirée de la quantité de cellules par ladite lyophilisation, tout en étant suffisante pour former une suspension aqueuse desdites cellules pour administration intra-respiratoire.

9. Procédé selon la revendication 8, caractérisé en ce que l'eau stérile ajoutée précitée contient une quantité efficace d'un agent mouillant pour favoriser la réhydratation des cellules précitées et leur implantation dans la muqueuse, ledit agent mouillant à la quantité présente n'inhibant pas la croissance desdites cellules.

10. Souche modifiée de *bordetella bronchiseptica* identifiée par souche ATCC n° 31437 ou souche qui en est dérivée et ayant essentiellement les mêmes caractéristiques pour un usage de vaccin intra-respiratoire, caractérisée en ce qu'elle est capable de se retirer des passages nasaux du porc et du chien après colonisation.

**Patentansprüche für die Vertragsstaaten: BE, CH, DE, FR, GB, IT, NL, SE**

1. Intrarespiratorischer Impfstoff für Tiere, die Infektionen durch Bordetella bronchiseptica erleiden können, dadurch gekennzeichnet, daß er aus einer wäßrigen Suspension lebensfähiger Zellen des als ATCC-Stamm Nr. 31437 identifizierten modifizierten Stammes von Bordetella bronchiseptica oder eines davon abgeleiteten Stammes mit im wesentlichen den gleichen Eigenschaften in bezug auf die Verwendbarkeit als intrarespiratorischer Impfstoff besteht und daß die genannte wäßrige Suspension mindestens $2 \times 10^3$ der genannten lebensfähigen Zellen in einem Milliliter enthält.

2. Impfstoff nach Anspruch 1, dadurch gekennzeichnet daß die genannte wäßrige Suspension mindestens $1 \times 10^5$ der genannten lebensfähigen Zellen in einem Milliliter und daneben noch Nährstoffreste aus der Kultur enthält.

3. Impfstoff nach Anspruch 1 oder 2, dadurch gekennzeichnet, daß die genannte wäßrige Suspension sprühbar ist und $1 \times 10^6$ bis $1 \times 10^8$ der genannten lebensfähigen Zellen in einem Milliliter enthält.

4. Impfstoff-Dosierform eines geschwächten, avirulenten Stammes von Bordetella bronchiseptica zur Besiedlung durch Beimpfung der Schleimhäute der Atemwege, dadurch gekennzeichnet, daß er aus einer Kombination aus einer ersten Ampulle, die gefriergetrocknete lebensfähige Zellen des B. bronchiseptica-Stammes der ATCC-Nr. 31437 oder eines davon abgeleiteten Stammes mit im wesentlichen den gleichen Eigenschaften in bezug auf die Verwendbarkeit als intrarespiratorischer Impfstoff enthält, wobei die in der genannten ersten Ampulle enthaltene Menge der Zellen mindestens eine Dosis des genannten Impfstoffs umfaßt, und einer zweiten Ampulle, die steriles Wasser für den Zusatz zu den Zellen in der genannten ersten Ampulle zur Bildung einer wäßrigen Suspension dieser Zellen enthält, besteht, wobei die genannte wäßrige Suspension, die durch Zusatz des genannten sterilen Wassers zu den genannten Zellen gebildet wird, eine Konzentration von mindestens $1 \times 10^6$ der genannten lebensfähigen Zellen in einem Milliliter besitzt.

5. Impfstoff-Dosierform nach Anspruch 4, dadurch gekennzeichnet, daß die zweite Ampulle in dem sterilen Wasser geiöst ein Netzmittel enthält und daß das genannte Netzmittel in einer wirksamen Menge vorliegt, die die Rehydratisierung der Zellen und ihre Einbettung in die Schleimhäute fördert, und nicht hemmend auf das Wachstum der genannten Zellen wirkt.

6. Impfstoff-Dosierform nach Anspruch 5, dadurch gekennzeichnet, daß das genannte Netzmittel ein anionisches oder ein nicht-ionisches Netzmittel ist.

7. Impfstoff-Dosierform nach Anspruch 4 bis 6, dadurch gekennzeichnet, daß die Menge des genannten sterilen Wassers in der genannten zweiten Ampulle ein Volumen von 40% bis 60% des Volumens des bei der Gefriertrocknung von den Zellen in der genannten ersten Ampulle entfernten Wassers besitzt, wobei die Menge der genannten lebensfähigen Zellen in der genannten ersten Ampulle eine Einzeldosis des genannten Impfstoffs in Höhe von $1 \times 10^6$ bis $1 \times 10^8$ Zellen umfaßt und die Menge des Wassers in der genannten zweiten Ampulle so festgelegt wird, daß sie zur Bildung der genannten wäßrigen Suspension mit einer Konzentration von $1 \times 10^6$ bis $1 \times 10^8$ lebensfähigen Zellen in einem Milliliter angemessen ist.

8. Verfahren zur Herstellung einer Impfstoff-Dosierform eines geschwächten avirulenten Stammes von Bordetella bronchiseptica zur Besiedlung durch Beimpfung der Schleimhäute der Atemwege, dadurch gekennzeichnet, daß eine Kultur des B. bronchiseptica-Stammes der ATCC-Nr. 31437 oder eines davon abgeleiteten Stammes mit im wesentlichen den gleichen Eigenschaften in bezug auf die Verwendbarkeit als intrarespiratorischer Impfstoff in einem wäßrigen Nährstoffmedium zur einer wäßrigen Suspension lebensfähiger Zellen mit einer Konzentration von mindestens $1 \times 10^7$ lebensfähigen Zellen in einem Milliliter kultiviert wird, daß eine wäßrige Lösung mikrobiologischer Kälteschutz und Stabilisierungsmittel zu der genannten Zellsuspension zugesetzt und dadurch eine Erniedrigung der Konzentration der Zellen verursacht wird, daß die verdünnte Zellsuspension der Gefriertrocknung unterworfen wird, wonach die erhaltenen gefriergetrockneten Zellen einen verminderten Gehalt an lebensfähigen Zellen infolge der durch die Gefriertrocknung bedingten Einbuße an Lebensfähigkeit besitzen, daß eine abgemessene Menge der genannten gefriergetrockneten Zellen in eine Impfstoff-Ampulle zur Lagerung und zum Transport des so hergestellten Impfstoffs abgefüllt wird, wobei die genannte Ampulle mindestens eine Dosis des genannten Impfstoffs enthält, und daß vor der Verabreichung des genannten Impfstoffs steriles Wasser zu der genannten Menge der Zellen hinzugefügt wird, wobei die Menge des genannten hinzuzufügenden Wassers um mindestens 25% geringer als das Volumen des von der genannten Zellmenge bei der genannten Gefriertrocknung entfernten Wassers, jedoch ausreichend ist zur Bildung einer wäßrigen Suspension der genannten Zellen für eine intrarespiratorische Verabreichung.

9. Verfahren nach Anspruch 8, dadurch gekennzeichnet, daß das genannte hinzuzufügende sterile Wasser eine wirksame Menge eines Netzmittels zur Förderung der Rehydratisierung der genannten Zellen und ihrer Einbettung in die Schleimhäute enthält, wobei das genannte Netzmittel in der vorliegenden Menge nicht hemmend auf das Wachstum der genannten Zellen wirkt.

10. Modifizierter Stamm von Bordetella bronchiseptica, der als ATCC-Stamm Nr. 31437 identifiziert ist, oder ein davon abgeleiteter Stamm mit im wesentlichen den gleichen Eigenschaften in bezug auf die Verwendbarkeit als intrarespiratorischer Impfstoff, dadurch gekennzeichnet, daß dieser Stamm nach der Besiedlung der Nasengänge von Schweinen und Hunden aus diesen Nasengängen vollständig wieder ausscheidbar ist.

**Claims for the Contracting State: AT**

1. A process for preparing an intra-respiratory vaccine for animals subject to *Bordetella bronchiseptica* infection, characterized in that viable cells of *Bordetella bronchiseptica* strain ATCC No. 31437 or of a strain derived therefrom and having essentially the same characteristics for intra-respiratory vaccine use are introduced into a suitable aqueous culture medium and said medium is incubated at a temperature favoring the growth of the organism.

2. The process of claim 1, wherein the cultured cells are harvested by conventional techniques and an aqueous suspension is prepared having at least $1 \times 10^5$ of viable cells per milliliter.

3. The process of claim 2 wherein an aqueous suspension is prepared which is sprayable and contains $1 \times 10^6$ to $1 \times 10^8$ of viable cells per milliliter.

4. A process of preparing a vaccine dose form of an attenuated avirulent strain of *Bordetella bronchiseptica* for implant colonization of the respiratory mucosa, characterized in that a culture of *B. bronchiseptica* strain ATCC No. 31437 or of a strain derived therefrom and having essentially the same characteristics for intra-respiratory vaccine use is cultured in an aqueous nutrient medium to obtain an aqueous suspension of viable cells having a concentration of at least $1 \times 10^7$ viable cells per milliliter, an aqueous solution of microbiological cryoprotectant and stabilizer means is added to said cell suspension with consequent dilution of the cell concentration, the diluted cell suspension is subjected to freeze-drying the resulting dried cells having a reduced content of viable cells because of the loss of viability resulting from freeze-drying, a measured quantity of said freeze-dried cells is introduced into a vaccine vial for storage and transport of the thus-prepared vaccine said vial containing at least one dose of said vaccine, and prior to administration of said vaccine sterile water is added to said quantity of cells the amount of said added water being at least 25% less than the volume of water removed from said cell quantity by said freeze-drying while being sufficient to form an aqueous suspension of said cells for intra-respiratory administration.

5. The process of claim 4, characterized in that said added sterile water contains an effective amount of a wetting agent for promoting the rehydration of said cells and the mucosal implanting thereof, said wetting agent in the amount present being non-inhibitory to the growth of said cells.

6. The process of claim 5, characterized in that said wetting agent is an anionic or non-ionic wetting agent.

7. The process of any one of claims 4 to 6, characterized in that said added sterile water is equal to 40 to 60% of the volume of water removed from said cell quantity by freeze-drying.

8. The process of any one of claims 4 to 7, characterized in that said vaccine vial contains from $1 \times 10^6$ to $1 \times 10^8$ of said viable cells per dose quantity.

### Revendications pour l'Etat contractant: AT

1. Procédé de préparation d'un vaccin intra-respiratoire pour des animaux sujets à une infection de *bordetella bronchiseptica*, caractérisé en ce que des cellules viables de souche ATCC n° 31437 de *bordetella bronchiseptica* ou d'une souche qui en est dérivée et ayant essentiellement les mêmes caractéristiques pour un usage comme vaccin intra-respiratoire sont introduites dans un milieu aqueux approprié de culture et en ce que ledit milieu est incubé à une température favorisant la croissance de l'organisme.

2. Procédé selon la revendication 1, caractérisé en ce que les cellules cultivées sont récoltées par des techniques traditionnelles et en ce qu'une suspension aqueuse est préparée, contenant au moins $1 \times 10^5$ cellules viables par millilitre.

3. Procédé selon la revendication 2, caractérisé en ce qu'une suspension aqueuse est préparée, qui est pulvérisable et contient $1 \times 10^6$ à $1 \times 10^8$ cellules viables par millilitre.

4. Procédé de préparation d'une forme de dose de vaccin d'une souche avirulente atténuée de *bordetella bronchiseptica* pour colonisation par implant de la muqueuse respiratoire caractérisé en ce qu'une culture de souche ATCC n° 31437 de *B. bronchiseptica* ou d'une souche qui en est dérivée et ayant essentiellement les mêmes caractéristiques pour un usage comme vaccin intra-respiratoire, est mise en culture dans un milieu nutritif aqueux pour obtenir une suspension aqueuse de cellules viables ayant une concentration d'au moins $1 \times 10^7$ cellules viables par millilitre, en ce qu'une solution aqueuse d'un moyen stabilisant et cryoprotecteur microbiologique est ajoutée à ladite suspension de cellules avec dilution conséquente de la concentration des cellules, en ce que ladite suspension diluée de cellules est soumise à une lyophilisation, les cellules séchées résultantes ayant une teneur réduite en cellules viables du fait de la perte de viabilité résultant de la lyophilisation, en ce qu'une quantité mesurée desdites cellules lyophilisées est introduite dans une fiole de vaccin pour stockage et transport du vaccin ainsi préparé, ladite fiole contenant au moins une dose dudit vaccin, et en ce qu'avant administration dudit vaccin, de l'eau stérile est ajoutée à ladite quantité de cellules, la quantité de ladite eau ajoutée étant d'au moins 25% de moins que le volume d'eau retirée de ladite quantité de cellules par la lyophilisation tout en étant suffisante pour former une suspension aqueuse desdites cellules pour une administration intra-respiratoire.

5. Procédé selon la revendication 4, caractérisé en ce que l'eau stérile ajoutée précitée contient une quantité efficace d'un agent mouillant pour favoriser la réhydratation des cellules précitées et leur implantation dans la muqueuse, ledit agent mouillant à la quantité présente n'inhibant pas la croissance desdites cellules.

6. Procédé selon la revendication 5, caractérisé en ce que l'agent mouillant précité est un agent mouillant anionique ou non-ionique.

7. Procédé selon l'une quelconque des revendications 4 à 6, caractérisé en ce que l'eau stérile ajoutée précitée représente 40 à 60% du volume d'eau retirée de la quantité précitée des cellules par la lyophilisation.

8. Procédé selon l'une quelconque des revendications 4 à 7, caractérisé en ce que la fiole précitée du vaccin contient de $1 \times 10^6$ à $1 \times 10^8$ cellules viables par quantité de dose.

### Patentansprüche für den Vertragsstaat: AT

1. Verfahren zur Herstellung eines intrarespiratorischen Impfstoffes für Tiere, die Infektionen durch Bordetella bronchiseptica erleiden können, dadurch gekennzeichnet, daß lebensfähige Zellen des B. bronchiseptica-Stammes der ATCC-Nr. 31437 oder eines davon abgeleiteten Stammes mit im wesentlichen den gleichen Eigenschaften in bezug auf die Verswendbarkeit als intrarespiratorischer Impfstoff in ein geeignetes wäßriges Kulturmedium eingebracht werden und das genannte Kulturmedium bei einer für das Wachstum des Organismus günstigen Temperatur inkubiert wird.

2. Verfahren nach Anspruch 1, dadurch gekennzeichnet, daß die kultivierten Zellen durch konventionelle Verfahren geerntet werden und daraus eine wäßrige Suspension mit mindestens $1 \times 10^5$ lebensfähigen Zellen in einem Milliliter hergestellt wird.

3. Verfahren nach Anspruch 2, dadurch gekennzeichnet, daß eine wäßrige Suspension hergestellt wird, die versprühbar ist und $1 \times 10^6$ bis $1 \times 10^8$ lebensfähige Zellen in einem Milliliter enthält.

4. Verfahren zur Herstellung einer Impfstoff-Dosierform eines geschwächten avirulenten Stammes von Bordetelle bronchiseptica zur Besiedlung durch Beimpfung der Schleimhäute der Atemwege, dadurch gekennzeichnet, daß eine Kultur des B. bronchiseptica-Stammes der ATCC-Nr. 31437 oder eines davon abgeleiteten Stammes mit im wesentlichen den gleichen Eigenschaften in bezug auf eine

**0012718**

Verwendbarkeit als intrarespiratorischer Impfstoff in einen wäßrigen Nährstoffmedium zu einer wäßrigen Suspension lebensfähiger Zellen mit einer Konzentration von mindestens $1 \times 10^7$ lebensfähigen Zellen in einem Milliliter kultiviert wird, daß eine wäßrige Lösung mikrobiologischer Kälteschutz und Stabilisierungsmittel zu der genannten Zellsuspension zugesetzt und dadurch eine Erniedrigung der Konzentration der Zellen verursacht wird, daß die verdünnte Zellsuspension der Gefriertrocknung unterworfen wird, wonach die erhaltenen gefriergetrockneten Zellen einen Verminderten Gehalt an lebensfähigen Zellen infolge der durch die Gefriertrocknung bedingten Einbuße an Lebensfähigkeit besitzen, daß eine abgemessene Menge der genannten gefriergetrockneten Zellen in eine Impfstoff-Ampulle zur Lagerung und zum Transport des so hergestellten Impfstoffs abgefüllt wird, wobei die genannte Ampulle mindestens eine Dosis des genannten Impfstoffes enthält, und daß vor der Verabreichung des genannten Impfstoffs steriles Wasser zu der genannten Menge der Zellen hinzugefügt wird, wobei die Menge des genannten hinzuzufügenden Wassers um mindestens 25% geringer als das Volumen des von der genannten Zellmenge bei der genannten Gefriertrocknung entfernten Wassers, jedoch ausreichend ist zur Bildung einer wäßrigen Suspension der genannten Zellen für eine intrarespiratorische Verabreichung.

5. Verfahren nach Anspruch 4, dadurch gekennzeichnet, daß das genannte hinzuzufügende sterile Wasser eine wirksame Menge eines Netzmittels zur Förderung der Rehydratisierung der genannten Zellen und ihrer Einbettung in die Schleimhäute enthält, wobei das genannte Netzmittel in der vorliegenden Menge nicht hemmend auf das Wachstum der genannten Zellen wirkt.

6. Verfahren nach Anspruch 5, dadurch gekennzeichnet, daß das genannte Netzmittel ein anionisches oder ein nicht-ionisches Netzmittel ist.

7. Verfahren nach Anspruch 4 bis 6, dadurch gekennzeichnet, daß die Menge des genannten hinzuzufügenden Wassers ein Volumen von 40% bis 60% des Volumens des bei der Gefriertrocknung von der genannten Zellmenge entfernten Wassers besitzt.

8. Verfahren nach Anspruch 4 bis 7, dadurch gekennzeichnet, daß die genannte Impfstoff-Ampulle $1 \times 10^6$ bis $1 \times 10^8$ der genannten lebensfähigen Zellen pro Dosismenge enthält.